(19) **Europäisches Patentamt**
**European Patent Office**
**Office européen des brevets**

(11) **EP 1 925 938 A1**

(12) **EUROPEAN PATENT APPLICATION**

(43) Date of publication:
**28.05.2008 Bulletin 2008/22**

(51) Int Cl.:
**G01N 33/542** *(2006.01)*     **G01N 33/50** *(2006.01)*
**G01N 33/68** *(2006.01)*

(21) Application number: **06024330.0**

(22) Date of filing: **23.11.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **PheneX Pharmaceuticals AG**
**67056 Ludwigshafen (DE)**

(72) Inventor: **The designation of the inventor has not yet been filed**

(74) Representative: **Bohmann, Armin K.**
**Bohmann & Loosen**
**Anwaltssozietät**
**Nymphenburger Strasse 1**
**80335 München (DE)**

Remarks:
A request for correction of the numbering of the first claim referred to as claim 32 such as to read "31" has been filed pursuant to Rule 88 EPC.

(54) **Method for determining the effect of a test agent on a target molecule**

(57) The present invention is related to a method for determining the effect of a test agent on a target molecule comprising the following steps:
a) providing the target molecule,
b) providing a target molecule interacting entity,
c) providing the test agent,
d) combining the target molecule, the test agent and the target molecule interacting entity,
whereupon the test agent induces a change in the conformational state of the target molecule and
said change in the conformational state of the target molecule results in a change in affinity of the target molecule for the target molecule interacting entity,

e) determining the amount of the target molecule interacting entity which interacts with the target molecule, or, alternatively, determining the amount of the target molecule which interacts with the interacting entity, and optionally
f) determining the effect of the test agent on the target protein,
whereby the target molecule is a protein from the family of nuclear receptors and either the target molecule or the target molecule interacting entity is immobilized, preferably immobilized to a surface .

**Description**

The present invention is related to a method for determining the effect of a test agent on a target molecule, a modulator for modulating the interaction between a target molecule and a target molecule interacting entity which can be identified by said method, the use of the method for the screening of a modulator for modulating the interaction between a target molecule and a target molecule interacting entity, and a method for the detection of a compound.

Current drug development is mostly based on identification and validation of target molecules and elucidation of the mode of action of drug candidates targeting the respective target molecule. Among the numerous targets known and in principle addressable by drugs, nuclear receptors are an important group thereof.

There are, in principle, two kinds of assays known to measure the effect of drug candidates, which are also referred to herein as test agents, on target molecules such as in particular, but not limited thereto, nuclear receptors, namely cellular assays and cell-free assays.

In most cellular assays addressing nuclear receptors, the effect of a receptor's activation on gene transcription is measured via a reporter gene, which expresses a detectable reporter gene such as, e.g., a luciferase, under the control of the nuclear receptor-dependent promoter. These assays are useful, but suffer from the typical disadvantages of cellular assays. More specifically, the assay procedure is lengthy, typically involving at least two days. Also when performing such assay, it is not clear whether the observed activation or inactivation of the receptor involves direct binding of the test compound to the receptor, or whether this is due to indirect effects. This "black box effect" has led in the past to the identification of compounds that were supposed to directly activate the receptor, but did in fact trigger a signal transduction cascade that led to an indirect activation of the reporter gene. Finally, toxic effects of high doses of the test substance will lead to an overall decrease in transcription, such that for many compounds the maximal testable concentrations are limited.

Cell-free assays for target molecules and for nuclear receptors in particular are advantageous insofar that they measure with high reliability the direct effects of the substances on the receptor. In most cases, the receptor proteins are purified from recombinant sources. The disadvantages of these assays are the followings. First, expression and purification of the receptor from recombinant sources in an active and responsive form is labour-intensive. Second, in some cases, such as the pharmacologically important glucocorticoid receptor, purification of the receptor leads to the loss of cofactors that are required to keep the receptor responsive, which leads to a complete loss of receptor activity. Thus, cell-free assays using purified protein are typically not possible. Apart from that, posttranslational modifications that occur in the natural cellular environment of the receptor and may be crucial to the functioning of the receptor, may be absent in preparations from recombinant sources. Finally, it is not possible to study the effect of extracellular stimuli, such as growth factor treatment, on the activity of the receptor in cell-free assays.

The problem underlying the present invention is thus to provide for a rapid method for determining the effect of a test agent on a target molecule. Another problem underlying the present invention is to provide a method which allows the immediate assignment of the observed reaction of the test system to the test agent. A further problem underlying the present invention is to provide for a method for determining the effect of a test agent on a target molecule, whereby the study of complex target molecules which may also require a cofactor for exerting their full activity, is possible in a cell-free assay. Finally, a problem underlying the present invention is to provide for a method for determining the effect of a test agent on a target molecule which overcomes the disadvantages of both cellular and cell-free assays, particularly those outlined above. A further problem underlying the present invention is to provide for a method for the detection of a compound in a sample.

These and other problems obvious to the one skilled in the art are solved by the subject matter of the attached independent claims. Preferred embodiments may be taken from the dependent claims.

More specifically, in a first aspect the problem underlying the present invention is solved by a method for determining the effect of a test agent on a target molecule comprising the following steps:

a) providing the target molecule,

b) providing a target molecule interacting entity,

c) providing the test agent,

d) combining the target molecule, the test agent and the target molecule interacting entity, whereupon the test agent induces a change in the conformational state of the target molecule and said change in the conformational state of the target molecule results in a change in affinity of the target molecule for the target molecule interacting entity,

e) determining the amount of the target molecule interacting entity which interacts with the target molecule, or, alternatively, determining the amount of the target molecule which interacts with the interacting entity, and optionally

f) determining the effect of the test agent on the target protein.

In an embodiment, the target molecule is a protein from the family of nuclear receptors.

In a further embodiment the target molecule or the target molecule interacting entity is immobilized, preferably immobilized to a surface .

In an embodiment in step d) a first amount of the target molecule interacting entity is interacting with the target molecule and a second amount of the target molecule interacting entity is not interacting with the target molecule, and wherein in step d) a first amount of the target molecule is interacting with the target molecule interacting entity, and a second amount of the target molecule is not interacting with the target molecule interacting entity.

In an embodiment either (a) the target molecule interacting entity which is interacting with the target molecule, is preferably separated from the target molecule interacting entity which is not interacting with the target molecule, or, alternatively, or (b) the target molecule which is interacting with the target molecule interacting entity, is preferabely separated from the target molecule which is not interacting with the target molecule interacting entity, preferably by immobilizing the target molecule interacting entity in case of (a) or, by immobilizing the target molecule in case of (b), whereby more preferably the support is a solid support.

In an embodiment the target molecule and/or the target molecule interacting entity are expression products expressed in a cell and the cell is lysed for providing a cell extract, for providing the target molecule and the target molecule interacting entity in steps a) and b), respectively.

In an embodiment the target molecule and/or the target molecule interacting entity is expressed in cellular extracts capable of performing *in vitro* translation reactions.

In a preferred embodiment the target molecule and the target molecule interacting molecule are expressed in the same cell and the cell is lysed for providing a cell extract for providing the target molecule and the target molecule interacting entity in step a) and b), respectively.

In an embodiment the test agent is added to the cell(s) prior to the lysis of the cell(s).

In an embodiment the test agent is added to the cell extract.

In a preferred embodiment the target molecule is an expression product expressed in a cell and the cell is lysed for providing a cell extract for providing the target molecule in step a) or the target molecule is an expression product expressed in a cellular extract capable of performing *in vitro* translation reactions.

In a further preferred embodiment the test agent is added to the cell extract.

In an embodiment the test agent is added to the cell.

In an embodiment the target molecule interacting entity is added to the cell extract.

In an embodiment the target molecule and/or the target molecule interacting molecule comprises a tag, whereby such tag preferably allows separation of the target molecule from a complex mixture.

In an embodiment the target molecule and/or the target molecule interacting entity comprises a tag, whereby such tag preferably allows separation of the target molecule interacting entity from a complex mixture.

In a preferred embodiment the tag is selected from the group comprising Protein A, more preferably a ZZ-domain, FLAG, hemagglutinin, His6, and an Ig sequence.

In a further preferred embodiment the tag is His6.

In an embodiment the target molecule and/or the target molecule interacting entity comprises a label, whereby such label preferably allows detection of the target molecule interacting entity and/or the detection of the target molecule.

In a preferred embodiment the label is selected from the group comprising enzymes, radioisotopes, fluorescent labels, luminescent labels, enzymatic labels, epitopes recognized by an antibody and biotin.

In a further preferred embodiment the label is luciferase or biotin in complex with streptavidin.

In an embodiment the target molecule interacting entity is a compound or fragment of a compound known to interact with the target molecule.

In an embodiment the target molecule is a protein, polypeptide or a peptide.

In an embodiment the target molecule is the liver X receptor alpha or the liver X receptor beta.

In a preferred embodiment the target molecule is the liver X receptor alpha in a heterodimeric complex with the retinoid X receptor alpha.

In an embodiment the target molecule is the aryl hydrocarbon receptor.

In an embodiment the target molecule interacting entity is selected from the group comprising proteins, polypeptides and peptides.

In a preferred embodiment the target molecule interacting entity is a synthetic peptide.

In a further preferred embodiment the synthetic peptide corresponds to or comprises the amino acid sequence of the cofactor interaction sequence found in nuclear receptor binding proteins.

In a more preferred embodiment the amino acid sequence of the cofactor interaction sequence comprises the amino acid sequence motif "LxxLL, wherein "L" is a hydrophobic amino acid preferably selected from the group comprising leucine, isoleucine and phenylalanine, and wherein "x" is any amino acid.

In an embodiment the target molecule interacting entity is selected from the group comprising proteins, polypeptides and peptides which are known to bind to nuclear receptors.

In an embodiment the target molecule interacting entity is a cofactor for the liver X receptor alpha and the liver X receptor beta, whereby the cofactor is preferably selected from the group comprising Tif2, SRC1, NcoA3, PGC1, RIP140, NCoR1 and SMRT1.

In an embodiment the target molecule interacting entity is a cofactor for the liver X receptor alpha and/or the liver X receptor beta, preferably selected from the group comprising Tif2, SRC1, NcoA3, PGC1, RIP140, NCoR1 and SMRT1.

In an embodiment the target molecule interacting entity is selected from the group comprising proteins binding to the aryl hydrocarbon receptor, preferably the target molecule interacting entity is ARNT.

In an embodiment the target molecule is a DNA-binding protein.

In a preferred embodiment the complex comprising the target molecule, the target molecule interacting entity and optionally the test agent, or the target molecule interacting entity which is interacting with the target molecule, is separated from complex mixtures, preferably the reaction mixture provided in step d) and more preferably from the target molecule interacting entity not interacting with the target molecule, by means of a nucleic acid immobilized to a support, preferably a solid support.

In a further preferred embodiment the nucleic acid molecule is a short double-stranded deoxyribonucleic acid which preferably comprises a motif recognized by the target molecule.

In an embodiment the complex comprising the target molecule, the target molecule interacting entity and optionally the test agent, or the target molecule interacting entity which is interacting with the target molecule, is separated from complex mixtures, preferably the reaction mixture provided in step d) and more preferably from the target molecule interacting entity not interacting with the target molecule, by means of a nucleic acid immobilized to a support, preferably a solid support.

In a preferred embodiment the nucleic acid is a double-stranded DNA molecule, which is recognised by the liver X receptor alpha and/or beta.

In a further preferred embodiment the nucleic acid molecule comprises at least a part of a gene, whereby the gene is selected from the group comprising the ABCA1 gene and-other genes which contain LXR responsive DNA elements within their sequence, preferably the nucleic acid molecule comprises the nucleic acid sequence GAA AGAAAT TTG ACC GAT AGT AAC CTC TGC GC.

In an embodiment the test agent is selected from the group comprising small molecules, antibodies, anticalines, aptamers, and spiegelmers

In a second aspect the problem underlying the present invention is solved by a modulator for modulating the interaction between the target molecule and the target molecule interaction entity which can be identified by a method according to the first aspect.

In a third aspect the problem underlying the present invention is solved by a method for the screening of a test agent which is a modulator for modulating the interaction between a target molecule and a target molecule interacting entity comprising the following steps:

a) providing the target molecule,

b) providing a target molecule interacting entity,

c) providing the test agent,

d) combining the target molecule, the test agent and the target molecule interacting entity,
whereupon the test agent induces a change in the conformational state of the target molecule and
said change in the conformational state of the target molecule results in a change in affinity of the target molecule
for the target molecule interacting entity,

e) determining the amount of the target molecule interacting entity which interacts with the target molecule, or,
alternatively, determining the amount of the target molecule which interacts with the interacting entity, and optionally

f) determining the effect of the test agent on the target protein.

In a fourth aspect the problem underlying the present invention is solved by the use of the method according to the first and/or third aspect for the screening of a modulator for modulating the interaction between the target molecule and the target molecule interaction entity.

In an embodiment of the fourth asepct in step c) the test agent is provided as a member of a library of test agents.

In an embodiment of the fourth asepct in step c) the test agent is provided as a plurality of members of a library of test agents.

In an embodiment of the fourth asepct the target molecule interacting entity which is interacting with the target molecule, is separated from the target molecule interacting entity which is not interacting with the target molecule, and/or from the other members of the library of test agents, or, alternatively, wherein the target molecule which is interacting with the target molecule interacting entity is separated from the target molecule which is not interacting with the target molecule interacting entity.

In a fifth aspect the problem underlying the present invention is solved by a method for determining the presence of a test agent which is suspected of being present in a test sample, comprising the following steps:

a) providing a target molecule,

b) providing a target molecule interacting entity,

c) providing a test sample which is suspected of comprising the test agent,

d) combining the target molecule, the test sample and the target molecule interacting entity,
whereupon the test agent, if it is present in the test sample induces a change in the conformational state of the target molecule and
said change in the conformational state of the target molecule results in a change in affinity of the target molecule for the target molecule interacting entity, and

e) determining the effect of the test sample on the target protein and more specifically on the complex formation between the target protein and the target protein interacting entity.

Apart from providing the advantage of solving the aforementioned problems, the method according to the present invention is particularly suitable to overcome the disadvantages of using cellular assays or cell-free assays for determining the effect of a test agent on a target molecule as outlined in the introductory part hereof. More specifically, the method of the present invention provides for a direct measurement of the effect of a test substance on the receptor's conformation, may use receptors from natural sources or receptors that are over-expressed in the natural environment, does not require purification of the receptor prior to the assay, is typically fast and can be performed, under standard conditions, within a period of three hours or less and is not sensitive to toxic effects of the substrate which allows to explore the full potency of the test agent without being limited to concentrations which are non-toxic to cells.

Without wishing to be bound by any theory, the method of the present invention measures the effect of a substance which is also referred to herein as a test agent, on the conformation of the target molecule such as a receptor and more specifically a nuclear receptors, via altered interactions of the target molecule with an entity interacting with the target molecule which is also referred to herein as target molecule interacting entity. In preferred embodiments such target molecule interacting entity is a cofactor of the target molecule, however, is not limited thereto. In such embodiment where the target molecule is a nuclear receptor the target molecule interacting entity is preferably a nuclear receptor cofactor. As preferably used herein a nuclear receptor cofactor is a protein that physically binds to nuclear receptors. A great number of such proteins has been described.

The following table contains some nuclear receptor cofactors, whereby the nuclear receptor cofactor which can be used in connection with the present invention are not limited thereto.

| Genbank Identifier | Name | Description |
|---|---|---|
| AAH07439.1 | SMIF | Smad interacting factor |
| AB002282 | MBF1alpha | multiprotein bridgeing factor1 |
| AF019415_2 | Nkx2.2 | homeobox protein |
| AF493978 | ERAP140 | estrogen receptor associated protein 140 |
| AK075223 | TRIP1 hom | unnamed protein product, putatively related to MSP1 PROTEIN |
| nageneseqAAA98244 | SM20 EGLN3 | hypothetical protein |
| NM_000177 | Gelsolin | gelsolin (amyloidosis, Finnish type) (GSN) |

(continued)

| Genbank Identifier | Name | Description |
|---|---|---|
| NM_000179 | MSH6 | mismatch repair homolgy protein |
| NM_000905 | NPY | neuropeptide Y |
| NM_000972 | L7/SPA | ribosomal protein L7a (RPL7A) |
| NM_001220 | CAM2 | calcium/calmodulin-dependent protein kinase (CaM kinase) II beta (CAMK2B) |
| NM_001328 | CTBP | C-terminal binding protein 1 (CTBP1) |
| NM_001450 | FHL2 | Homo sapiens four and a half LIM domains 2 (FHL2) |
| NM_001455 | FKHRL1 | forkhead box 03A (FOXO3A) |
| NM_001607 | EC2.3.16 | peroxisomal, 3-oxoacyl-Coenzyme A thiolase (ACAA1) |
| NM_001948 | DUT fl | dUTP pyrophosphatase |
| NM_002065 | GLUL | glutamate-ammonia ligase (glutamine synthase) |
| NM_002805 | TRIP1 | proteasome (prosome, macropain) 26S subunit, ATPase, 5 |
| NM_003345 | UBE2 1 | ubiquitin-conjugating enzyme E2I (UBC9 homolog, yeast) |
| NM_003489 | RIP140 | nuclear receptor interacting protein 1 (NRIP1) |
| NM_003563 | SPOP | speckle-type POZ protein |
| NM_003592 | Cul1 | cullin 1 |
| NM_003743 | SRC1 | nuclear receptor coactivator 1 (NCOA1) |
| NM_003884 | pCAF | p300/CBP-associated factor (PCAF) |
| NM_003950 | PAR4 | preapoptotic response gene 4 |
| NM_004229 | DRIP150 | cofactor required for Sp1 transcriptional activation, subunit 2 (150kD) (CRSP2) |
| NM_004236 | ALIEN | thyroid receptor interacting protein 15 (TRIP15) |
| NM_004290 | ARA54 | androgen receptor associated protein 54 |
| NM_004380 | CBP | CREB binding protein (Rubinstein-Taybi syndrome) |
| NM_004726 | POB1 | RALBP1 associated Eps domain containing 2 (REPS2), variant |
| NM_004774 | TRAP220 | PPAR binding protein (PPARBP), frame shifted, only up to aa 195 TRAP220 sequence |
| NM_004890 | PRG1 hom | PRG1 homologue, sperm associated antigen 7 |
| NM_004902 | CAPER | coactivator of activating protein-1 and estrogen receptors |
| NM_005066 | PSF | splicing factor proline/glutamine rich (polypyrimidine |
| NM_005144 | hairless | hairless protein isoform a |
| NM_005359 | SMAD4com | SMA-AND MAD-RELATED PROTEIN 4 |
| NM_005388 | PHLP | phosducin-like (PDCL) |
| NM_005412* | SHMT2 | frame shift*, likely to encode artificial LXXLL peptide, from pos nt195 of Ref.Seq Entry Reisolate |
| NM_005437 | ARA70 | nuclear receptor coactivator 4 |
| NM_005902 | SMAD3com | SMA- AND MAD-RELATED PROTEIN 3 |
| NM_006292 | TSG101 | tumor susceptibility gene 101 (TSG101) |
| NM_006311 | NCoR1 | nuclear receptor co-repressor 1 |

(continued)

| Genbank Identifier | Name | Description |
|---|---|---|
| NM_006312 | SMRT | nuclear receptor co-repressor 2 *, has 75 aa deletion from aa 2322 onwards |
| NM_006333 | SunCoR | nuclear DNA-binding protein (C1D) |
| NM_006337 | MIF1 | microspherule protein 1 (MCRS1) |
| NM_006384 | CIB | calcium and integrin binding 1 (calmyrin) |
| NM_006388 | TIP60 | HIV-1 Tat interactive protein, 60 kD (HTATIP) |
| NM_006503 | MIP224 | proteasome (prosome, macropain) 26S subunit, ATPase, 4 |
| NM_006534 | SRC3 | nuclear receptor coactivator 3 (NCOA3) |
| NM_006540 | TIF2 | nuclear receptor coactivator 2 (NCOA2) |
| NM_006579 | EBP emopamil BP | emopamil binding protein (sterol isomerse) |
| NM_006744 | RBP | retinol binding protein 4, plasma (RBP4) Reisolate |
| NM_006813 | PNRC | proline rich nuclear receptor coactivator |
| NM_006837 | JAB1 | Jun activation domain binding protein |
| NM_007056 | SWAP2 | suppressor of white apricot homolog 2 |
| NM_007114 | ARA160 | TATA element modulatory factor |
| NM_007273 | REA | repressor of estrogen receptor activity |
| NM_007317 | KID-like | kinesin-like protein 4 Reisolate |
| NM_012245 | NCoA62 | SKI-interacting protein (SNW1) |
| NM_012295 | Cabin1 | calcineurin binding protein 1 |
| NM_013261 | PGC1 | peroxisome proliferative activated receptor, gamma, coactivator 1 (PPARGC1) |
| NM_014071 | RAP250 | nuclear receptor coactivator 6 (NCOA6) |
| NM_014288 | NRIF3 | integrin beta 3 binding protein |
| NM_014958 | KIAA0915 | guanine nucleotide exchange factor (GEF) 15 (ARHGEF15) |
| NM_015001 | SHARP | SMART/HDAC1 associated repressor protein, receptor interaction domain |
| NM_015927 | ARA55 | transforming growth factor beta 1 induced transcript 1 |
| NM_016556 | GT198 | TBP-1 interacting protein |
| NM_017761 | PNRC2 | hypothetical protein FLJ20312 , starts upstream of ATG |
| NM_017814 | AK000429 | hypothetical protein FLJ20422 |
| NM_020967 | CIA | nuclear receptor coactivator 5 (NCOA5) |
| NM_021245 | Calsarcin 2 | myozenin 1 |
| NM_021969 | SHP | nuclear receptor subfamily, group B, member 2 (NR0B2) |
| NM_022455 | ARA267-frag1 | NUCLEAR RECEPTOR-BINDING Su-var |
| NM_024309 | ABIN2 | A20-binding inhibitor of NF-kappaB activation-2 |
| NM_025082 | ERaip | hypothetical protein FLJ13111 |
| NM_025112 | NR39BP3 | hypothetical protein |
| NM_080546 | CTL1 | choline transporter like protein |
| NM_138923 | TAFII250 | TATA box binding protein (TBP)-associated factor, 250 kD (TAF1) |

In contrast to the methods known in the art, the target molecule - target molecule interacting entity complexes are not dependent on the purification of the receptor. Instead, the test agent is added either to the cells expressing the target molecule and/or the target molecule interacting entity shortly before lysis or to a crude cell lysate of said cells. Complexes between the target molecule and the target molecule interacting entity are then already existing or allowed to form. In a further step the complex is preferably detected via immobilization of either the target molecule or the target molecule interacting entity on a support, preferably a solid support, whereupon either the target molecule or the target molecule interacting entity is specifically detected.

As used herein, the target molecule and the target molecule interacting entity are collectively referred to as the interacting molecules.

As preferably used herein, the target molecule can be any molecule which is capable of interacting with another molecule which is also referred to herein as target molecule interacting entity. More preferably, the target molecule interacting entity and the target molecule are a pair of molecules which interact with each other under physiological conditions. However, such interaction is not limited to the prevalence of such physiological conditions. Physiological conditions are preferably those conditions existing in cells, tissues, organs and organisms, respectively, typically at a temperature between about 32 and 43 °C, more preferably 36 to 41 °C and most preferably at 37 °C. Even more preferably, such interaction is an interaction as present in any chemical, biochemical, immunological or enzymatic reaction typically performed under physiological conditions. It is also within the present invention that such interaction is the interaction of two molecules or more molecules in a biological system. As preferably used insofar, a biological system is a cell, either eukaryotic or prokaryotic, a tissue, an organ or an organism.

The target molecule and the target molecule interacting entity, respectively, are thus not limited to a specific type of compound as long as the afore-mentioned relationship and interaction, respectively, is in principle possible. Accordingly, the target molecule and the target molecule interacting entity, respectively, i.e. the interacting molecules can each and individually and independently from each other be a protein, a polypeptide, or a peptide, or a fragment thereof. It is also within the present invention that the interacting molecules are each and individually and independently from each other a be lipid, a nucleic acid molecule or a carbohydrate, whereby the carbohydrate is preferably a polymer based on one or several carbohydrate moieties, or any combination of the afore-mentioned types or classes of molecules. Finally, said interacting molecules can each and individually and independently from each other be a small molecule, an antibody, an anticaline, an aptamer or a spiegelmer as preferably defined herein.

As preferably used herein, a protein is a chain of amino acids which are linked via a peptidic bond preferably involving 40 or more amino acids. Proteins are synthesized via the translation of an RNA molecule on a ribosome or may be chemically synthesized.

As preferably used herein, a polypeptide is a chain of amino acids which are linked via a peptidic bond preferably involving more than two amino acids, independently of the origin of the polypeptide. A polypeptide can, in an embodiment, be a protein or a peptide.

As preferably used herein, a peptide is a chain of amino acids which are linked via a peptidic bond preferably involving 39 or less amino acids. Peptides can be synthesized via the translation of an RNA molecule on a ribosome, via the cleavage of short fragments from a larger protein, via enzymatic synthesis in a cell, or via chemical synthesis.

As preferably used herein, a lipid is a hydrophobic substance of one of the following types: a fatty acids, esters of fatty acids such as triglycerides, a glycerophospholipid, a sphingolipid, an isopreonid, or a steroid.

As preferably used herein, a carbohydrate is a molecule that contains oxygen, hydrogen, and carbon atoms have the general chemical formula $C_n(H_2O)_n$ or a derivative of such, whereby n is preferably from 1 to 200. A carbohydrate may also contain other elements such as sulfur or nitrogen, but these are usually minor components. Carbohydrates consist of monosaccharide sugars, which can be polymerised to varying chain lengths.

As preferably used herein, a nucleic acid molecule is a polymer of nucleotides, such as ribonucleic acid or deoxyribonucleic acid, of a length greater than two nucleotides.

As preferably used herein, an antibody is a protein which has domains that adopt the three dimensional structure typical for immunoglobulins, and which has at least an antigen binding site which can bind to a certain chemical structure (s) with high affinity. Antibodies can consist of varying amounts of immunoglobulin domains, for example, single-chain Fv fragments consist of two immunoglobuline domains, whereas a human immunoglobulin G can consist of twelve immunoglobuline domains. Antibodies can be of natural origin, or can be generated *in vitro* by methods such as phage display-based methods, but are not limited thereto.

As preferably used herein, a small molecule is a molecule with a mass of lower than 5000, preferably lower than 2000, and more preferably lower than 1000 dalton, with no limitation on the composition or structure of the molecule. Molecules with this mass include drug-like molecules, which can be used for derivatisation with the aim to designing molecules that can be used as therapeutics.

As preferably used herein, a peptide aptamer which is also referred to herein as target binding polypeptide, may be generated by using methods according to the state of the art such as phage display. Basically, a library of peptide is generated, such as in form of phages, and this kind of libraries is contacted with the target molecule. Those peptides binding to the target molecule are subsequently removed, preferably as a complex with the target molecule, from the respective reaction. It is known to the one skilled in the art that the binding characteristics, at least to a certain extent, depend on the particularly realized experimental set-up such as the salt concentration and the like. After separating those peptides binding to the target molecule with a higher affinity or a bigger force, from the non-binding members of the library, and optionally also after removal of the target molecule from the complex of target molecule and peptide, the respective peptide(s) may subsequently be characterised. Prior to the characterisation optionally an amplification step is realized such as, e. g. by propagating the peptide coding phages. The characterisation preferably comprises the sequencing of the target binding peptides. Basically, the peptides are not limited in their lengths, however, preferably peptides having a lengths from about 8 to 20 amino acids are preferably obtained in the respective methods. The size of the libraries may be about $10^2$ to $10^{18}$, preferably $10^8$ to $10^{15}$ different peptides, however, is not limited thereto.

As preferably used herein, an anticaline is a particular form of target binding polypeptides are which are, among others, described in German patent application DE 197 42 706.

As preferably used herein, an aptamer is a D-nucleic acid which is either single stranded or double stranded and which specifically interacts with a target molecule. The manufacture or selection of aptamers is, e. g., described in European patent EP 0 533 838. Basically the following steps are realized. First, a mixture of nucleic acids, i. e. potential aptamers, is provided whereby each nucleic acid typically comprises a segment of several, preferably at least eight subsequent randomised nucleotides. This mixture is subsequently contacted with the target molecule whereby the nucleic acid(s) bind(s) to the target molecule, such as based on an increased affinity towards the target or with a bigger force thereto, compared to the candidate mixture. The binding nucleic acid(s) are/is subsequently separated from the remainder of the mixture. Optionally, the thus obtained nucleic acid(s) is amplified using, e. g., polymerase chain reaction. These steps may be repeated several times giving at the end a mixture having an increased ratio of nucleic acids specifically binding to the target from which the final binding nucleic acid is then optionally selected. These specifically binding nucleic acid(s) are referred to aptamers. It is obvious that at any stage of the method for the generation or identification of the aptamers samples of the mixture of individual nucleic acids may be taken to determine the sequence thereof using standard techniques. It is within the present invention that the aptamers may be stabilized such as, e. g., by introducing defined chemical groups which are known to the one skilled in the art of generating aptamers. Such modification may for example reside in the introduction of an amino group at the 2'-position of the sugar moiety of the nucleotides.

As preferably used herein, a spiegelmer is a particular form of aptamers which consists, at least essentially, of L-nucleotides rather than D-nucleotides as aptamers. The generation of spiegelmers is described in international patent application WO 98/08856. Spiegelmers are characterized by the fact that they have a very high stability in biological system and, comparable to aptamers, specifically interact with the target molecule against which they are directed. In the process of generating spiegelmers, a heterogonous population of D-nucleic acids is created and this population is contacted with the optical antipode of the target molecule. Subsequently, those D-nucleic acids are separated which do not interact with the optical antipode of the target molecule. But those D-nucleic acids interacting with the optical antipode of the target molecule are separated, optionally determined and/or sequenced and subsequently the corresponding L-nucleic acids are synthesized based on the nucleic acid sequence information obtained from the D-nucleic acids. These L-nucleic acids which are identical in terms of sequence with the aforementioned D-nucleic acids interacting with the optical antipode of the target molecule, will specifically interact with the naturally occurring target molecule rather than with the optical antipode thereof. Similar to the method for the generation of aptamers it is also possible to repeat the various steps several times and thus to enrich those nucleic acids specifically interacting with the optical antipode of the target molecule.

As preferably used herein, the test agent is any compound the effect of which on a target molecule is to be determined. Such test agent can be any kind of agent, including those classes of compounds specified in connection with the target molecule and target molecule interacting entity, respectively.

Both the target molecule and the target molecule interacting entity can be derived from natural sources, recombinant sources or be provided by chemical synthesis. Suitable natural sources are tissue cultures or tissue culture cells. Recombinant sources are preferably transfected tissue culture cells, cells infected with a recombinant virus, in vitro translation mixes or genetically modified low organisms including, but not limited to, yeast, fungi or prokaryotes. Finally, chemical synthesis is a means to provide said target molecule and target molecule interacting entity, respectively. Depending on the chemical nature, the one skilled in the art will be aware of respective methods and means to provide the very same. The very same applies also to the test agent.

A particularly preferred source for the target molecule and target molecule interacting entity are transfected tissue culture cells. The more preferred tissue culture cells are those which are known to be involved in human and animal diseases, respectively, such as HEK293 cells, HeLa cells, fibroblast cells, HepG2 cells, THP1 cells, and MCF1 cells. The means for preparing such transfected tissue culture cells are known to the one skilled in the art and respective methods are, for example, described in Current Protocols in Cell Biology (Wiley InterScience, October 2006, ISBN: 0-471-24108-3, Chapter 20). In connection with the preparation of the target molecule and target molecule interacting entity, respectively, it will be acknowledged by the ones skilled in the art, that they can, however, there is no need insofar, be purified or partially purified from the cellular extract prepared from such tissue culture cells, prior to the assay. It is within the present invention that the target molecule, the test agent and the target molecule interacting entity are combined, whereupon the test agent induces a change in the conformational state of the target molecule and said change in the conformational state of the target molecule results in a change in affinity of the target molecule for the target molecule interacting entity. The combining of these three compounds can be performed under conditions where all three compounds are present in solution or under conditions where either the target molecule or the target molecule interacting entity is immobilized.

The immobilization typically occurs by using a means that binds with high affinity to the target molecule or the target molecule interacting entity, preferably to part thereof, which is also referred to herein as affinity reagent. Such part thereof may be a part of the target molecule itself, or the target molecule interacting entity itself, such as, but not limited to, a domain or epitope, or may have been added to the target molecule or the target molecule interacting entity. Such part added to the target molecule or the target molecule interacting entity is also referred to herein as a tag and will be discussed in more detail herein later.

A particular group of target molecules or target molecule interacting entities is one that, due to its inherent binding characteristics, defines a possible affinity reagent in itself, i.e. does not require a tag as used herein for immobilization. One example of the respective specific group of target molecules or target molecule interacting entities are target molecules or target molecule interacting entities having a DNA-binding domain which can be captured by binding to a cognate DNA binding site. The DNA binding site characteristics define the characteristics of the nucleic acid used as an affinity reagent. For example, if the target molecule or target molecule interacting entity is a nuclear receptor or other form of transcription factor, the affinity reagent will preferably be a short natural DNA fragment or a double-stranded synthetic oligomer.

Irrespective of the particularities of the immobilization step as such, the support to which either the target molecule or the target molecule interacting entity is coated or modified such that the affinity reagent is comprised or contained in and/or on said support. Such support is preferably a solid support as known to the one skilled in the art and comprises, but is not limited to, beads, resins or the wells of, for example, ELISA plates, typically in the 16, 96 or 384 well plate format. Preferred materials for the beads are sepharose, agarose, latex, and magnetic materials, preferred resins are organic polymers, such as cross-linked polystyrene.

As obvious from the disclosure herein, it is within the present invention that either the target molecule or the target molecule interacting entity is immobilized. Accordingly, any step or measure described in connection with the immobilization and subsequent steps such as, including but not limited to, detection described herein for the target molecule applies also for the target molecule binding entity and vice versa.

In the immobilization step the non-specific binding sites are blocked as known to one skilled in the art. Preferred blocking agents are known to the one skilled in the art and, comprise, for example, solutions of bovine serum albumine, fat free milk protein, casein, and serum.

As a further step in the method for determining the effect of a test agent on a target molecule according to the present invention, in the combining of the target molecule and the target molecule interacting entity as well as the test agent, complex formation takes place. Such complex formation can either occur under conditions where all the components are unimmobilised or where at least one, more preferably, the target molecule or the target molecule interacting entity are immobilised as outlined above.

In *in vivo* complex formation, the test agent is added to the cells before the cells are lysed, whereby the cells can be cells which either individually code for and thus provide the target molecule and the target molecule interacting entity, or the cells harbour the genetic information for both, i.e. coding for and thus providing both the target molecule and the target molecule interacting entity. The cells are lysed by means known to the one skilled in the art. Such means comprise, among others, the use of detergents such as Triton X-100, NP-40. Tween 20, sodium dodecyl sulfate, deoxycholate, and others, the use of hypertonic or hypotonic conditions, the use of mechanical stress such as shearing forces, the use of ultrasound, or a combination of any of said methods. Under such conditions, complex formation can take place before immobilisation.

Alternatively, the complex formation can occur *in vitro*. Under such circumstances, the test agent is added to the extract containing the target molecule and the target molecule interacting entity, or the reaction containing both the target molecule and the target molecule interacting entity. In such case, complex formation and immobilisation can take place in the same reaction, under the proviso that the target molecule and target molecule interacting entity are contained in a vessel providing the support to which any one of the compounds involved may be and factually is immobilised. In case different cells are coding for the target molecule and the target molecule interacting entity, the extracts prepared from said different cells are mixed subsequently and then used in step d) of the method according to the present invention. It will also be acknowledged by the ones skilled in the art that the combining of step d) of the method of the present invention can be performed by allowing any of the compounds provided in steps a) to c) in various combinations to react, i. e. combining the target molecule and the target molecule interacting entity, combining the target molecule and the test agent, combining the target molecule interacting entity and the test agent as well as combining all three of them, i.e. the target molecule, the target molecule interacting entity and the test agent. Such combining can occur either in the presence or absence of the support which preferably provides the surface for immobilizing either the target molecule or the target molecule interacting entity.

It will be acknowledged by the ones skilled in the art that in the formation of the complex comprising the target molecule and the target molecule interacting entity and preferably also the test agent, further compounds may be added to take part in said complex formation; these further compounds can be part of the endogenous environment of the cells producing the target molecule and the target molecule interacting entity, respectively, or can be added exogenously. Such compounds can also be used to "bridge" interactions between the target molecule and the target molecule interacting entity. Such further compounds can be of any of the classes of compounds, preferably of any class of compound as defined herein in connection with the target molecule and the target molecule interacting entity, respectively.

Typically after step d) step e) of the method of the present invention, the unbound material is removed from the reaction. Typically this occurs by washing procedures known to the ones skilled in the art. Typically, the washing procedures are such that the complex formed in step d) are not dissolved or, if so, only to a known extent or an extent which can be determined by the ones skilled in the art.

In step e) of the method of the present invention the amount of the target molecule interacting entity which interacts with the target molecule, or, alternatively, the amout of the target molecule which interacts with the target molecule interacting entity is determined. An affinity reagent that binds with high affinity to the target molecule or to a part thereof, or, respectively to the target molecule interacting entity or to a part thereof, may be used for such purpose. Such part can also be a tag as defined herein, whereby such part or tag mediates the interaction between the target molecule interacting entity and the affinity means, whereby the affinity means as such is ultimately detected. For the detection of this kind of affinity means methods and means are known in the art and comprise, among others, labels suitable for detection as also described herein, in particular in connection with the labelling of the target molecule interacting entity. Alternatively, such part can also be artificially added to the target molecule interacting entity and preferably comprises labels known to the one skilled in the art.

Preferred labels for detection are polypeptide tags that are attached to proteins, or small compounds which are covalently attached to the protein. Preferred labels include the following ones: fluorescent proteins (Muller-Taubenberger A. Application of fluorescent protein tags as reporters in live-cell imaging studies. Methods Mol Biol. 2006; 346: 229-46.), luciferases such as firefly, gaussia and renilla luciferases *(*Hastings JW, Morin JG. Photons for reporting molecular events: green fluorescent protein and four luciferase systems. Methods Biochem Anal. 2006;47:15-38., *and* Wilson T, Hastings JW. Bioluminescence. Annu Rev Cell Dev Biol. 1998;14:197-230.*),* other enzymes that can catalyze reactions which can be used in colorimetric, fluorimetric or luminometric read-outs, such as alkaline phosphatase or horse-radish peroxidase, or other oxido-reductases. In addition, all affinity tags can be employed for detection of the attached protein via labelled reagents that bind with sufficient affinity to the affinity tag.

It will be acknowleged by the ones skilled in the art that any affinity reagent as known to the ones skilled in the art can be used for the detection of the target molecule interacting entity contained in the complex comprising the target molecule, the target molecule interacting entity and the test agent, or the target molecule, under the proviso that the non-bound target molecule interacting entities have been washed away and, preferably, that the target molecule is immobilised to the support. However, the affinity reagent can also be directed to the immobilised target molecule if the complex formation being part of the method of the present invention results in an altered binding characteristic of such affinity reagent. This prerequisite can easily be checked by a person skilled in the art using routine experiments such as, e.g. competition assays.

For the immobilisation of either the target molecule or the target molecule interacting entity, preferably a tag is used. Perferably such tag allows the separation of the respective compound, i. e. the target molecule or the target molecule interacting entity or a complex thereof which may optionally also contain the test agent, from a complex mixture. Preferably, such complex mixture is the reaction as described in or resulting from step d) of the method according to the present invention. Preferably, such tag is known to the one skilled in the art and is either a polypeptide that is covalently attached to a protein of interest, or a small molecule such as biotin or fluorescein which is covalently attached to proteins of interest. These polypetides or small molecules bind with high affinity to other reagents (e.g. the ZZ domain of Staphylococcus aureus protein A bind to the Fc part of an immuneglobulin G, biotin binds to avidin or streptavidin, fluorescein binds to an anti-fluorescein antibody), which can be used to detect or purify the protein or any molecule of interest.

For a polypeptide that is covalently attached to a protein or any molecule of interest, and which facilitates the purification and/or detection of the protein or any molecule of interest, typically, the attachment of a tag, in case the molecule of interest in a protein, is brought about by the expression of the protein of interest from DNA - constructs in which the sequence coding for the protein of interest is attached to a sequence coding for the tag in the same reading frame, either at the 5' end (i.e. the amino terminus of the encoded protein) or the 3' end (i.e. the carboxy-terminus of the encoded protein).

Commonly used tags include the following ones: glutathione-S-transferase, maltose-binding protein, NusA, thioredoxin, ubiquitin, BAP, poly(His), STREP, chitin-binding protein, S-tag, short antibody epitopes (e.g. Myc, FLAG, VSV, HA), and others (see also Waugh DS. Making the most of affinity tags. Trends Biotechnol. 2005; 23:316-20, and Lichty JJ, Malecki JL, Agnew HD, Michelson-Horowitz DJ, Tan S. Comparison of affinity tags for protein purification.Protein Expr Purif. 2005; 41:98-105.).

It will be acknowledged by the ones skilled in the art that for both immobilization and detection purposes, a tag is functional only in combination with a complementary compound which specifically interacts with such tag. The functions of both the tag and the complementary compound specifically interacting with such tag, are interchangeable. This means that the tag, particularly for immobilization purposes, is part of the molecule to be immobilizes and the complementary compound is part of the support, or the complementary compound is part of the molecule to be immobilized and the tag is part of the support. Preferred combinations of tag and complementary compounds are the followings: The ZZ-domain of protein A and the Fc-part of an immuneglobulin, glutathione and glutathione-S-transferase, biotin and avidin, biotin and streptavidin, maltose and maltose-binding protein, the myc - epitope and an antibody recognising said eptitope.

A preferred tag is IgG, whereby the IgG is part of the component of the reaction as of step d) which is to be immobilised, i. e. either the target molecule or the target molecule interacting entity. The support then comprises the ZZ-domain of protein A from Staphylococcus aureus, which is known to bind to the Fc-part of IgG, so as to interact with the IgG moiety of the component of the reaction as of step d) to be immobilized. Of course, and in accordance what has been said above, the orientation may be the other way round, i. e. that the protein A is attached to the compound to be immobilised, i. e. the target molecule or the target molecule interacting entity, and the IgG is coated to the support, preferably the ELISA plates. In a preferred alternative, such tag is an immobilised DNA fragments that will bind with high affinity to the nuclear receptor. In this case, the complementary compound is not necessary as the function of the complementary compound is inherent to the nuclear receptor due to its characteristic to bind to DNA fragments.

The detection of the target molecule and the target molecule interacting entity, respectively, can occur as outlined above using the affinity reagents. Alternatively, the respective compound can also be provided with or already inherently contain a label allowing the detection of said compound. Particularly preferred labels are those known to the one skilled in the art.

It is most preferred to have a fusion protein with an enzymatically active protein or protein domaine such as luciferase, horse-radish peroxidase or alkaline phosphatase or other reporter enzymes known to the one skilled in the art providing such label. These enzymes or enzymatic activities are attached to at least one of the compounds of reaction d) of the method according to the present invention, more specifically the one which is to be detected, namely either the target molecule or the target molecule interacting entity. Particularly preferred fusion proteins are those with luciferase from Gaussia or Renilla. Reagents suitable for these enzymatic activities are known to the ones skilled in the art and are preferably the following ones: Coelenterazin, D-[4,5 Dihydro-2-(6-hydroxy-2-benzothiazolyl)-4-thiazolecarboxylic acid], for use with Renilla Luciferase, and Luciferin, L-[4,5 Dihydro-2-(6-hydroxy-2-benzothiazolyl)-4-thiazole carboxylic acid] for use with Firefly Luciferase.

The method according to the present invention may also be used as a screening method, i. e. a method for identifying a compound which interacts with the complex formation between a target molecule and a targe molecule interacting entity. Such method comprises the steps of

a) providing the target molecule,

b) providing a target molecule interacting entity,

c) providing the test agent,

d) combining the target molecule, the test agent and the target molecule interacting entity,
whereupon the testing agent induces a change in the conformational state of the target molecule and
said change in the conformational state of the target molecule results in a change in affinity of the target molecule for the target molecule interacting entity,

e) determining the effect of the test agent on the target protein and more specifically on the complex formation between the target protein and the target protein interacting entity.

The steps and means used for carrying out such steps in connection with such screening method are the same as described above for the method according to the present invention for determining the effect of the test agent on a target molecule. It will be acknowledged that the test agent is preferably a member of a library of molecules and may be either used individually or in combination with other members of the library in such screening method.

In a further aspect, the various steps of the method for determining the effect of a test agent can be used to detect both in a quantitative and qualitative manner the presence of a test agent. For such purpose, after step d) of the method of the present invention for determining the effect of a test agent on a target molecule the step is such that from the amount of the complex formed between the target molecule and the target molecule interacting entity the presence and the amount of the present test agent can be determined. A particularly preferred assay insofar is described in the example part and the individual steps taken insofar are obvious to the one skilled in the art.

Such method preferably comprises the steps of

a) providing a target molecule,

b) providing a target molecule interacting entity,

c) providing a test sample,

d) combining the target molecule, the test sample and the target molecule interacting entity, whereupon the analytes present in the test sample may induces a change in the conformational state of the target molecule and said change in the conformational state of the target molecule results in a change in affinity of the target molecule for the target molecule interacting entity,

e) determining the effect of the test sample on the target protein and more specifically on the complex formation between the target protein and the target protein interacting entity.

In such method the test agent is preferably a compound the presence and/or concentration of which in a sample has to be determined. Such samples include, but are not limited to blood, liquor, urine, tear, saliva, sperma, vaginal secrete and other sample known and used in the medicinal art. It will, however, be acknowledged that the sample may also be any sample such as an environmental sample or a sample taken from a laboratory environment such as fermentation broth, cell culture or tissue culture.

From step e) the presence and/or amount of the test agent can be determined, whereby such determination typically involved the use of a standard curve. It is within the skills of the person skilled in the art the prepare such standard curve. Preferably such standard curve is established by using in the above specified method one or several known amount of the test agent or the to be test agent and determine the effect thereof. Comparing the effect seen when running the method using a sample to be analyzed with the standard curve allows the determination of the presence and in particular the amount of the test agent in the reaction and thus in the test sample.

The present invention is now further illustrated by the examples from which further advantages, features and embodiments may be taken.

More specifically,

Fig. 1 is a schematic representing the principal steps of the method according to the present invention;

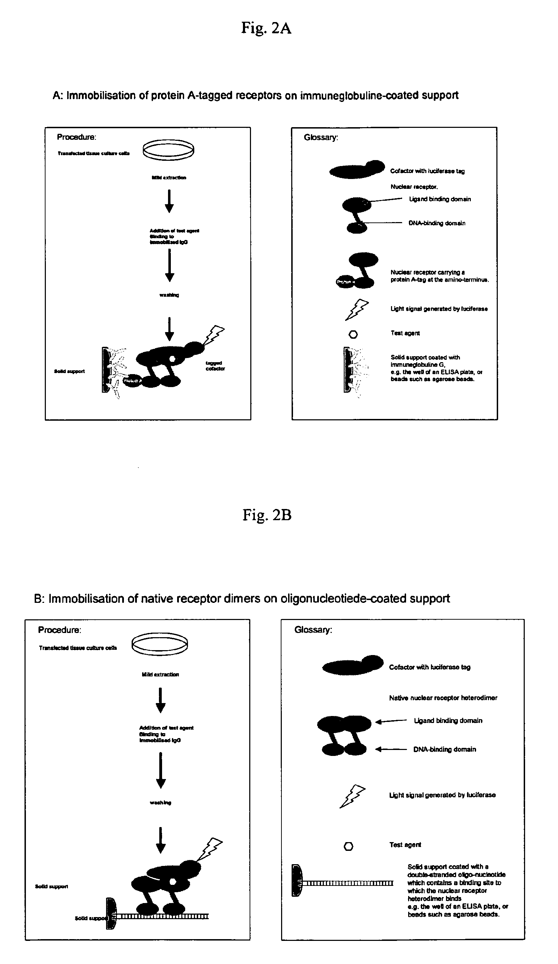

Fig. 2 is a schematic representing an embodiment of the present invention wherein the target molecule is a receptor, more specifically a heterodimeric nuclear receptor, and the target molecule interacting entity is the luciferase-tagged cofactor of the nuclear receptor; the nuclear receptor is immobilized to a surface either through a protein A tag of the receptor with the surface being coated with IgG (Fig.2 (A)), or through its DNA-binding domain binding to a nucleic acid molecule being attached to the surface and containing a sequence specifically interacting with the DNA-binding domain (Fig. 2(B));

Fig. 3 is a diagram indicating luciferase activity as a function of the test agent in a method according to the present invention using LXRalpha as a target molecule and Tif2 (amino acids 548 to 878) as a target molecule interacting entity;

Fig. 4 is a diagram indicating luciferase activity as a function of the test agent in a method according to the present invention using LXRalpha as a target molecule and TRAP220 (amino acids 433-803) as a target molecule interacting entity; and

Fig. 5 is a diagram indicating the amount of target molecule interacting entity bound to the target molecule under various experimental conditions.

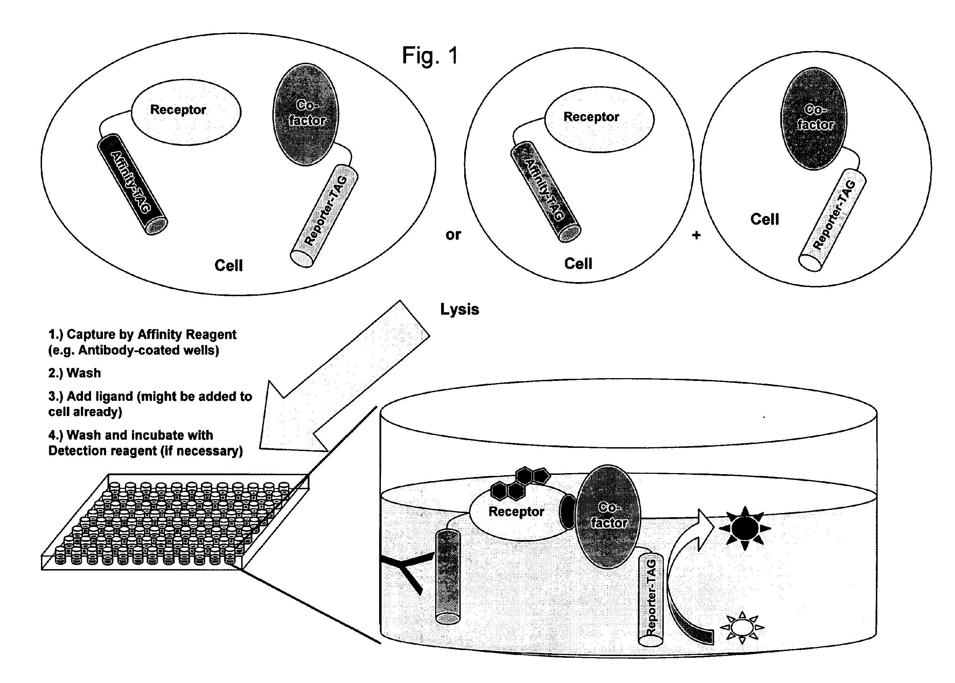

Fig. 1 is a schematic representing the principal steps of the method in accordance with an embodiment of the present invention. More specifically, the target molecule and the target molecule interacting entity are produced in either the same or a different cell. The target molecule comprises a tag which is an affinity tag. The affinity tag is, e.g., but not limited thereto, a protein A tag. The target molecule interacting entity comprises either a label or a tag. In the illustrated embodiment the target molecule interacting entity is a label which allows the detection of the target molecule interacting entity. A preferred label is an enzyme activity such as a luciferase as such label can be produced by a cell. The cell(s) is/are lysed, preferably under mild conditions, and the extract thereof is used further in the method. Optionally, such extract may be subject to a purification step.

The extracts are added to a reaction vessel. Alternatively, the extracts are obtained in the cell culture means such as tissue culture flasks where the cell(s) has/have been cultivated. The reaction vessel is in the depicted embodiment a 96 well plate. A surface of or contained in the reaction vessel is coated with a compound which is interacting, preferably specifically interacting, with the affinity tag attached to the target protein. Such compound is typically an IgG interacting with the protein A tag. Other combinations consisting of a distinct tag and such compound specifically interacting therewith, are known to the ones skilled in the art. The surface which is coated with such compound is either the surface of the reaction vessel or a surface contained in the reaction vessel. Such surface contained in the reaction vessel is, e.g., provided by one or several beads contained in the reaction vessel. Such beads are known to the ones skilled in the art.

Once the tag of the target protein has interacted with the compound which is coated to the surface, and is thus immobilized to said surface the target molecule interacting entity is added to the reaction. Alternatively, the target molecule interacting entity is added to the reaction at the same time or prior to the immobilization of the target molecule to the compound which is coated to the surface, preferably immobilized through the tag attached to the target molecule. In either case, an immobilized complex consisting of the target molecule and the target molecule interacting entity is formed. The immobilized complex is washed so as to remove any unbound or uncomplexed target molecule and/or unbound or uncomplexed target molecule interacting entity. This washing step allows that the tag or label attached to the target molecule interacting entity is removed from the reaction thus reducing the background signal or background noise arising from non-bound target molecule and/or the non-bound target molecule interacting entity.

In case the target molecule and the target molecule interacting entity are added separately or consecutively to the reaction, it is also within the present invention that after each step with which either the target molecule or the target molecule interacting entity is added to the reaction, a washing step is performed.

The test agent may be added prior or after the immobilization of the target molecule to the surface. Further the test agent may be added prior or after the formation of the complex between the target molecule and the target molecule interacting entity.

Figs 2A and 2B illustrate an embodiment of the method of the present invention. More specifically, a nuclear receptor is used as the target molecule. The receptor is shown as a heterodimeric protein, as for example the heterodimer of the liver X receptor alpha and the retinoid X receptor alpha.

In Fig. 2A, the nuclear receptor is immobilised via a protein A tag, and immuneglobuline G coated to the surface of a solid support. In Fig. 2B, a native nuclear receptor heterodimer (containing no tag) is immobilised via a double-stranded oligonucleotide bound to a solid support. The oligonucleotide contains a sequence which as a high-affinity binding site for the DNA-binding domain of the nuclear receptor dimer. In both cases, the nuclear receptor proteins and the luciferase -tagged cofactor are expressed in mammalian cells (e.g. HEK 293 cells). The test agent is added to extracts prepared from those cells, and the mixture is allowed to bind to the solid support. After unbound cofactor has been removed by washing of the solid support, the relative amount of cofactor binding to the immobilised nuclear receptor can be determined via the activity of the luciferase tag.

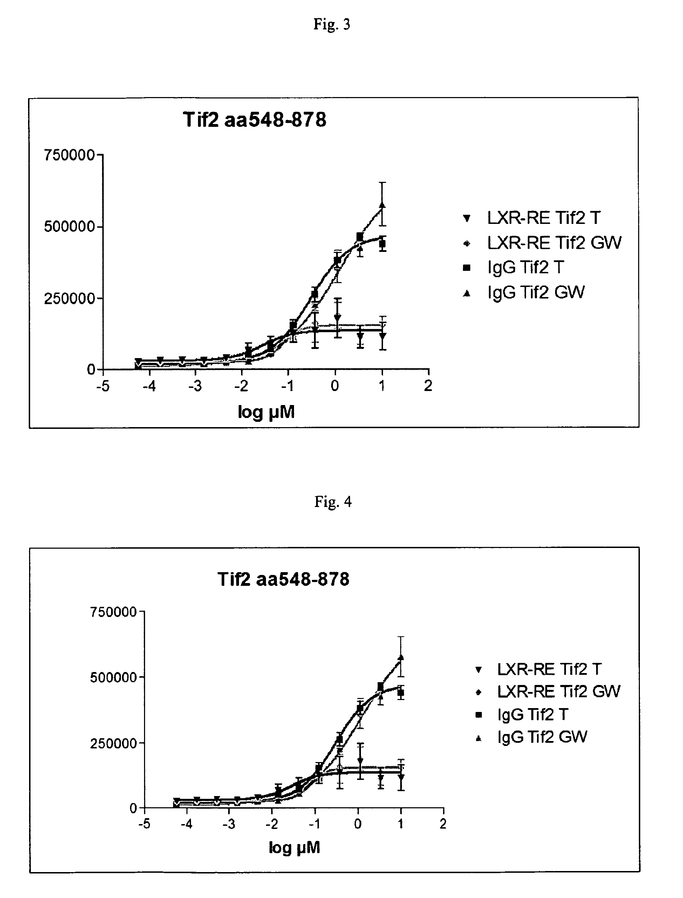

The subject matter of Figs. 3 to 5 will be explained in more detail in the example part herein.

**Example 1: Assay using LXRalpha and Tif2**

Target molecule: human LXRalpha (NR1H3), full-length protein, expressed from as a fusion protein with the ZZ domain of Protein A, the ZZ domain being at the amino terminus

Target molecule interacting molecule: human Tif2 (aa548 - 878), expressed as a fusion protein with Renilla Luciferase. Renilla Luciferase is at the amino terminus

**Materials:**

**Test agents:**

T0901317: availble from Sigma-Aldrich corporation, number T2320

GW3965 : availble from Sigma-Aldrich corporation G6295

Both compounds are described in Albers M, Blume B, Schlueter T, Wright MB, Kober I, Kremoser C, Deuschle U, Koegl M. A novel principle for partial agonism of liver X receptor ligands. Competitive recruitment of activators and repressors. J Biol Chem. 2006;281:4920-30.

**Plasmids**

Expression constructs have been generated using standard methods of DNA manipulation.
For the expression of Protein A tagged proteins, DNA coding for the ZZ-domain of Protein A has been inserted before the attL site into pTREX dest30 using standard methods. This vector allows for the expression of fusion proteins which carry the Renilla luciferase protein fused to their amino terminus.
For generation of an expression vector for fusion proteins with Renilla luciferase, pRluc-C2 (Packard-Bioscience) was digested with NdeI and XbaI and the CMV-Rluc NdeI/XbaI fragment gelpurified and ligated into NdeI/XbaI digested pcDNA3 (Invitrogen). The resulting plasmid was named pcDNA3-Rluc. The GATEWAY-cassette A was cloned behind the Rluc as an XhoI-XbaI fragment using the primers GW1XhoI GATCGCCTCGAGACAAGTTTGTACAAAAAAGCTGAACG and GW2XbaI GATCGCTCTAGAATCACCACTTTGTACAAGAAAG for the amplification of the GATEWAY cassette A.
For both plasmids, inserts can be transferred into the vectors using the GATEWAY recombinational cloning system (Invitrogen, Carlsbad).

**Plates**

Nunc 436110 96 Well Immuno Platten, white (LumiNunc), F96, MaxiSorb IgG
Sigma 15006-100MG IgG from rabbit serum

**Streptavidin**

Unconjugated streptavidin, CE0301C from Cortex Biochem, USA

**Lipofectamine 2000**

from Invitrogen (Carlsbad, USA)

**PBS: phosphate buffered saline**

| | |
|---|---|
| NaCl | 8 g |
| KCl | 0,2 g |
| $Na_2HPO4$ | 1,44 g |
| $KH_2PO4$ | 0,24 g |
| Dissolve in 800 ml $dH_2O$ | |
| Adjust pH to 7.4 with HCl | |

**Renilla assay buffer:**

1,1M NaCl
2,2 mM Na2EDTA (EDTA pH8.0)
220 mM KxPO4 pH 5,1
0,44 mg/ml Bovine Serum Albumine
1,3 mM NaN3
2,5 $\mu$M coelenterazine

Lysis buffer:

**12,5% glycerol**
Roche complete inhibitor mix of protease inhibitors
10mM dithiothreitol
0,1% Tween 20
137mM NaCl
10 mM Phosphate
2,7 mM KCl
pH to 7.4 with HCl

**Medium (for HEK293 cells)**

MEM +Earle's, -Glutamine, Gibco 21090
Additives:

10% FCS
1x Pen/Strep (Gibco)
0,1mM Non-essential amino acids
2mM glutamine
1mM Na-pyruvate

**OptiMEM I:**

From Gibco, # 31985-047

**Coating of microtiter plates**

**IgG coating:**

Plates were coated overnight at 4°C with 100μl per well of a 0,02μg/μl solution of rabbit IgG in PBS.
PBS was poured off, then plates were incubated with 300μl of 5% non-fat dry milk in PBS for 1 hour to block non-specific binding sites.
Plates were washed three times with 200μl PBS.
PBS was poured off the plates, plates were sealed with adhesive plastic foil, and stored at 4°C for no longer than two weeks.

**Coating with an oligonucleotide**

Plates were coated overnight at 4°C with 100μl per well of a 0,02μg/μl solution of streptavidin in PBS.
Plates were washed once with 200μl PBS.
100μl of a 250nM solution of a double-stranded oligo with biotin covalently attached to the 5' end was added. This oligo had the follwowing sequence, which is bound with high affinity by a heterodimer of LXRalpha and RXRalpha: GAAAGAAATTTGACCGATAGTAACCTCTGCGC.
The biotinylated oligo was allowed to bind to the immobilised streptavidin for one hour at room temperature.
The solution was poured off, and plates were incubated with 300μl of 5% non-fat dry milk in PBS for 1 hour to block non-specific binding sites.
Plates were washed three times with 200μl PBS.
PBS was poured off the plates, plates were sealed with adhesive plastic foil, and stored at 4°C for no longer than two weeks.

**Transfection:**

The target molecule and the target molecule interacting entity were separately transfected into HEK 293 cells on cell culture dishes with a diameter of 10cm as follows:

Cells: HEK 293 cells

10 million cells per 10cm dish were seeded in 10ml medium

1ml OptiMEM were mixed with 20μl of Lipofectamine 2000

1ml OptiMEM were mixed with 6μg of DNA per transfected construct

The Lipofectamine and DNA mixes were combined and incubated for 30 minutes at room temperature, before the mix was added to the HEK 293 cells.
Transiently transfected cells were grown for 44 hours before lysis

**Lysis of the cells**

All steps were done at 4°C. All buffers were pre-cooled on ice. The centrifuge was pre-cooled to 4°C.
Medium was removed from the transfected cells, and cells were washed once with 5ml PBS.
300μl lysis buffer was added, and cells were scraped off the plate in lysis buffer and put in a 1,5 ml plastic tube, vortexed for 5 seconds, and incubated for 10 minutes on ice.
Lysates were centrifuged 2 minutes at 13.000 rpm in a tabletop centrifuge.
The supernatant was collected.

**Nuclear Receptor activity assays**

Lysates were diluted 1:5 in PBS 5mM DTT
The diluted lysates from the transfection of the target molecule (LXRalpha/RXRalpha) and the target molecule interacting entity (Tif2) were mixed 1:1.
50μl of this mix was added to 24 wells of an IgG-coated microtiter plate, and to 24 wells of a microtiter plate coated with an oligonucleotide with a high affintiy for the LXRalpha/RXRalpha heterodimer.
The test agents were serially diluted with 1+2 dilution steps in DMSO with a starting concentration of 200μM. This results in the following concentrations (in μM) (before addition to the lysates):
200 66,7 22,2 7,4 2,5 0,82 0,27 0,09 0,03 0,01 0,003 0,001
This dilution series was subsequently used in assays in duplicate in both the IgG-coated and the oligonucleotide-coated microtiter plates, as follows:

The test agent was added to the lysates in the wells by transferring 2,5μl of the dilution series into the wells with the lysates, to yield a 10 fold dilution of the test agent in the lysate. The lysates and the test agent were mixed by gentle shaking for 5 seconds.

The plates with lysates and test agent were incubated for 2 hours on ice.
All subsequent steps were performed at room temperature.
The wells with lysates and test agent were washed 4 times with 200μl PBS, and 80μl PBS was added after the last wash.
Activity was measured on a standard plate luminometer. Reactions were started by injection of 70μl Renilla assay buffer into the wells, and measuring time was 2 seconds. Measurements were done at room temperature.

**Result:**

The result is shown in Fig. 3 as a dose-response curve. The x-axis represents the micromolar concentration of the test agent in a logarithmic representation. The y-axis represents the measured luciferase activiy (arbitrary units). The mean of the duplicates is shown, the deviation from the mean is indicated by error bars.
The origin of the curves are indicated in the legend:

LXR-RE: results from microtiter plates coated with oligonucleotides

Tif2: the target molecule interacting entity is Tif2 (amino acids 548 to 878)

IgG: results from microtiter plates coated with oligonucleotides

T: the compound is T0901317

GW: the compound is GW3965

**Example 2: Assay using LXRalpha and TRAP220**

The experiment was done exactly the same as described in example 1, except that the target molecule interacting entity was not Tif2, but TRAP220, amino acids 433-803.

The result is shown in Fig. 4. For an explanation of the legend, see example 1.

**Example 3: Method for determining the amount of target molecule interacting entity bound to the target molecule**

The experiment was done exactly the same as described in example 1, with the following differences:

The target molecules used were human estrogen receptor alpha, and human estrogen receptor beta, as indicated in the figure.

The target molecule interacting molecules were the protein encoded by the mRNA in the Genbank entry accession number BC015202, and the cofactor PGC1 (human).

The test agent was estradiol. Only a single concentration was tested.

The target molecule and the target molecule interacting entity were co-transfected, and estradiol had been added to the cells 30 minutes prior to cell lysis to a final concentration of $10\mu M$.

Only IgG-coated plates were used.

In this experiment, the Y axis represents the percentage of the cofactor employed in the assay that had bound to the estrogen receptor. This was determined as follows:

The luciferase activity in the lysates containing the Renilla-tagged cofactors was determined by measuring $50\mu l$ of 1:1000 dilution of the lysate in PBS in a luminometer as described in example 1. The luciferase activity of the input material was then calculated from that result, and the percentage bound was calculated as follows:

$$\text{Percentage bound} = \text{input luciferase activity} / \text{bound luciferase activity} \ \times \ 100.$$

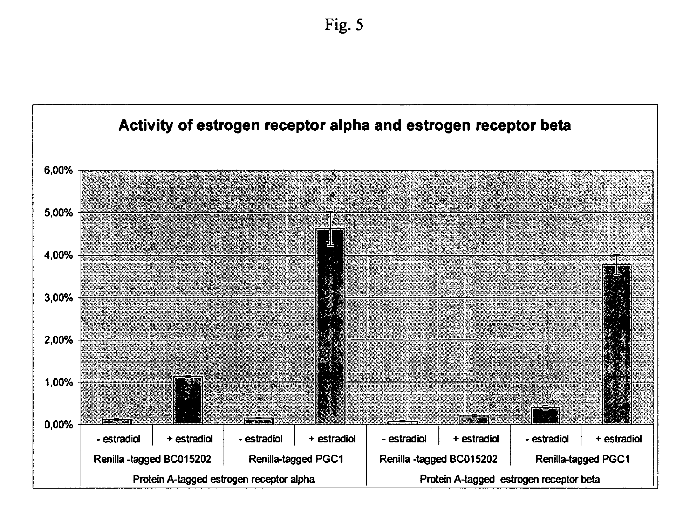

Results from triplicates are shown in Fig. 5, error bars are standard deviations.

It will be obvious to anyone skilled in the art that this kind of assay also amends itself to the measurement of dose-response curves, as in the examples 1 and 2.

**Example 4: Method for the detection of compounds activating the aryl-hydrocarbon receptor, such as naphto-flavones**

The experiment was done exactly the same as described in example 1, with the following differences:

The target molecule was the aryl hydrocarbon receptor (AhR, the dioxin receptor).

The target molecule interacting interacting entity is the aryl hydrocarbon receptor nuclear transclocator (ARNT).

The target molecule and the target molecule interacting molecule were expressed in the same cells.

The test entity was beta-naphto-flavone.

The test entity was added to the cells one hour prior to lysis to a final concentration of $20\mu M$.

The features of the present invention disclosed in the specification, the claims and/or the drawings may both separately and in any combination thereof be material for realizing the invention in various forms thereof.

**Claims**

1. A method for determining the effect of a test agent on a target molecule comprising the following steps:

a) providing the target molecule,

b) providing a target molecule interacting entity,

c) providing the test agent,

d) combining the target molecule, the test agent and the target molecule interacting entity, whereupon the test agent induces a change in the conformational state of the target molecule and said change in the conformational state of the target molecule results in a change in affinity of the target molecule for the target molecule interacting entity,

e) determining the amount of the target molecule interacting entity which interacts with the target molecule, or, alternatively, determining the amount of the target molecule which interacts with the interacting entity, and optionally

f) determining the effect of the test agent on the target protein,

whereby the target molecule is a protein from the family of nuclear receptors and either the target molecule or the target molecule interacting entity is immobilized, preferably immobilized to a surface.

**2.** The method according to claim 1, wherein in step d) a first amount of the target molecule interacting entity is interacting with the target molecule and a second amount of the target molecule interacting entity is not interacting with the target molecule, and wherein in step d) a first amount of the target molecule is interacting with the target molecule interacting entity, and a second amount of the target molecule is not interacting with the target molecule interacting entity.

**3.** The method according to claim 1 or 2, wherein either (a) the target molecule interacting entity which is interacting with the target molecule is preferably separated from the target molecule interacting entity which is not interacting with the target molecule, or, alternatively, or (b) the target molecule which is interacting with the target molecule interacting entity, is preferably separated from the target molecule which is not interacting with the target molecule interacting entity, preferably by immobilizing the target molecule interacting entity in case of (a) or, by immobilizing the target molecule in case of (b), whereby more preferably the support is a solid support.

**4.** The method according to any of claims 1 to 3, wherein the target molecule and/or the target molecule interacting entity are expression products expressed in a cell and the cell is lysed for providing a cell extract, for providing the target molecule and the target molecule interacting entity in steps a) and b), respectively.

**5.** The method according to any of claims 1 to 4, wherein the target molecule and/or the target molecule interacting entity is expressed in cellular extracts capable of performing *in vitro* translation reactions.

**6.** The method according to claim 4, wherein the target molecule and the target molecule interacting molecule are expressed in the same cell and the cell is lysed for providing a cell extract for providing the target molecule and the target molecule interacting entity in step a) and b), respectively.

**7.** The method according to any of claims 4 or 6, wherein the test agent is added to the cell(s) prior to the lysis of the cell(s).

**8.** The method according to any of claim 4 to 6, wherein the test agent is added to the cell extract.

**9.** The method according to claim 4, wherein the target molecule is an expression product expressed in a cell and the cell is lysed for providing a cell extract for providing the target molecule in step a) or the target molecule is an expression product expressed in a cellular extract capable of performing *in vitro* translation reactions.

**10.** The method according to claim 9, wherein the test agent is added to the cell extract.

**11.** The method according to claim 9, wherein the test agent is added to the cell.

**12.** The method according to any of claims 9 or 10, wherein the target molecule interacting entity is added to the cell extract.

**13.** The method according to any of claims 1 to 12, wherein the target molecule and/or the target molecule interacting molecule comprises a tag, whereby such tag preferably allows separation of the target molecule from a complex mixture.

**14.** The method according to any of claims 1 to 13, preferably according to any of claims 1 to 12, wherein the target molecule and/or the target molecule interacting entity comprises a tag, whereby such tag preferably allows separation of the target molecule interacting entity from a complex mixture.

**15.** The method according to claim 13 or 14, whereby the tag is selected from the group comprising Protein A (ZZ-domain), FLAG, hemagglutinin, His6, and an Ig sequence.

**16.** The method according to claim 15, wherein the tag is His6.

**17.** The method according to any of claims 1 to 16, wherein the target molecule and/or the target molecule interacting entity comprises a label, whereby such label preferably allows detection of the target molecule interacting entity and/or the detection of the target molecule.

**18.** The method according to claim 17, wherein the label is selected from the group comprising enzymes, radioisotopes, fluorescent labels, luminescent labels, enzymatic labels, epitopes recognized by an antibody and biotin.

**19.** The method according to claim 18, wherein the label is luciferase or biotin in complex with streptavidin.

**20.** The method according to any of claims 1 to 19, wherein the target molecule interacting entity is a compound or fragment of a compound known to interact with the target molecule.

**21.** The method according to any of claims 1 to 20, wherein the target molecule is a protein, polypeptide or a peptide.

**22.** The method according to claim 1-21, wherein the target molecule is the liver X receptor alpha or the liver X receptor beta.

**23.** The method according to claim 22, wherein the target molecule is the liver X receptor alpha in a heterodimeric complex with the retinoid X receptor alpha.

**24.** The method according any of claims 1 to 21, wherein the target molecule is the aryl hydrocarbon receptor.

**25.** The method according to any of claims 1 to 24, wherein the target molecule interacting entity is selected from the group comprising proteins, polypeptides and peptides.

**26.** The method according to claim 25, wherein the target molecule interacting entity is a synthetic peptide.

**27.** The method according to claim 26, wherein the synthetic peptide corresponds to or comprises the amino acid sequence of the cofactor interaction sequence found in nuclear receptor binding proteins.

**28.** The method according to claim 27, wherein the amino acid sequence of the cofactor interaction sequence comprises the amino acid sequence motif "LxxLL, wherein "L" is a hydrophobic amino acid preferably selected from the group comprising leucine, isoleucine and phenylalanine, and wherein "x" is any amino acid.

**29.** The method according to any of claims 1 to 28, preferably according to claim 1-21, wherein the target molecule interacting entity is selected from the group comprising proteins, polypeptides and peptides which are known to bind to nuclear receptors.

**30.** The method according to any of claims 1 to 29, preferably claim 22, wherein the target molecule interacting entity is a cofactor for the liver X receptor alpha and the liver X receptor beta, whereby the cofactor is preferably selected from the group comprising Tif2, SRC1, NcoA3, PGC1, RIP140, NCoR1 and SMRT1.

**32.** The method according to any of claims 1 to 29, preferably claim 23, wherein the target molecule interacting entity is a cofactor for the liver X receptor alpha and/or the liver X receptor beta, preferably selected from the group comprising Tif2, SRC1, NcoA3, PGC1, RIP140, NCoR1 and SMRT1.

**32.** The method according to any of claims 1 to 31, preferably claim 24, wherein the target molecule interacting entity is selected from the group comprising proteins binding to the aryl hydrocarbon receptor, preferably the target molecule interacting entity is ARNT.

**33.** The method according to any of claims 1 to 32, wherein the target molecule is a DNA-binding protein.

**34.** The method according to claim 33, wherein the complex comprising the target molecule, the target molecule interacting entity and optionally the test agent, or the target molecule interacting entity which is interacting with the target molecule, is separated from complex mixtures, preferably the reaction mixture provided in step d) and more preferably from the target molecule interacting entity not interacting with the target molecule, by means of a nucleic acid immobilized to a support, preferably a solid support.

**35.** The method according to claim 34, wherein the nucleic acid molecule is a short double-stranded deoxyribonucleic acid which preferably comprises a motif recognized by the target molecule.

**36.** The method according to any of claims 1 to 35, preferably any of claims 22, 23, 30, 31, and 32, wherein the complex comprising the target molecule, the target molecule interacting entity and optionally the test agent, or the target molecule interacting entity which is interacting with the target molecule, is separated from complex mixtures, preferably the reaction mixture provided in step d) and more preferably from the target molecule interacting entity not interacting with the target molecule, by means of a nucleic acid immobilized to a support, preferably a solid support.

**37.** The method according to claim 36, wherein the nucleic acid is a double-stranded DNA molecule, which is recognised by the liver X receptor alpha and/or beta.

**38.** The method according to claim 37, wherein the nucleic acid molecule comprises at least a part of a gene, whereby the gene is selected from the group comprising the ABCA1 gene and other genes which contain LXR responsive DNA elements within their sequence, preferably the nucleic acid molecule comprises the nucleic acid sequence GAA AGAAAT TTG ACC GAT AGT AAC CTC TGC GC.

**39.** The method according to any of claims 1 to 38, wherein the test agent is selected from the group comprising small molecules, antibodies, anticalines, aptamers, and spiegelmers

**40.** A modulator for modulating the interaction between the target molecule and the target molecule interaction entity which can be identified by a method according to any of the preceding claims.

**41.** A method for the screening of a test agent which is a modulator for modulating the interaction between a target molecule and a target molecule interacting entity comprising the following steps:

a) providing the target molecule,
b) providing a target molecule interacting entity,
c) providing the test agent,
d) combining the target molecule, the test agent and the target molecule interacting entity,
whereupon the test agent induces a change in the conformational state of the target molecule and
said change in the conformational state of the target molecule results in a change in affinity of the target molecule for the target molecule interacting entity,
e) determining the amount of the target molecule interacting entity which interacts with the target molecule, or, alternatively, determining the amount of the target molecule which interacts with the interacting entity, and optionally
f) determining the effect of the test agent on the target protein.

**42.** Use of the method according to any of the preceding claims for the screening of a modulator for modulating the interaction between the target molecule and the target molecule interaction entity.

**43.** Use according to claim 42, wherein in step c) the test agent is provided as a member of a library of test agents.

**44.** Use according to claim 42 or 43, wherein in step c) the test agent is provided as a plurality of members of a library of test agents.

**45.** Use according to claim 43 and 44, wherein the target molecule interacting entity which is interacting with the target molecule, is separated from the target molecule interacting entity which is not interacting with the target molecule, and/or from the other members of the library of test agents, or, alternatively, wherein the target molecule which is interacting with the target molecule interacting entity is separated from the target molecule which is not interacting with the target molecule interacting entity.

**46.** A method for determining the presence of a test agent which is suspected of being present in a test sample, comprising the following steps:

a) providing a target molecule,
b) providing a target molecule interacting entity,
c) providing a test sample which is suspected of comprising the test agent,
d) combining the target molecule, the test sample and the target molecule interacting entity,
whereupon the test agent, if it is present in the test sample induces a change in the conformational state of the target molecule and
said change in the conformational state of the target molecule results in a change in affinity of the target molecule for the target molecule interacting entity, and
e) determining the effect of the test sample on the target protein and more specifically on the complex formation between the target protein and the target protein interacting entity.

Fig. 1

Fig. 2A

A: Immobilisation of protein A-tagged receptors on immuneglobuline-coated support

Fig. 2B

B: Immobilisation of native receptor dimers on oligonucleotiede-coated support

Fig. 3

Fig. 4

Fig. 5

**Activity of estrogen receptor alpha and estrogen receptor beta**

| | - estradiol | + estradiol | - estradiol | + estradiol | - estradiol | + estradiol | - estradiol | + estradiol |
|---|---|---|---|---|---|---|---|---|
| | Renilla -tagged BC015202 | | Renilla-tagged PGC1 | | Renilla -tagged BC015202 | | Renilla-tagged PGC1 | |
| | Protein A-tagged estrogen receptor alpha | | | | Protein A-tagged estrogen receptor beta | | | |

**European Patent**
**Office**

**PARTIAL EUROPEAN SEARCH REPORT**     Application Number

which under Rule 45 of the European Patent Convention EP 06 02 4330
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/058532 A (ASTRAZENECA AB [SE]; BAMBERG KRISTER [SE]; NEBB HILDE [NO]) 1 August 2002 (2002-08-01) * the whole document * In particular: p. 1, l. 4-8; p. 4, l. 11 - p. 5, l. 16; p. 8. l. 6-9; claims 26-29 ----- | 1-46 | INV. G01N33/542 G01N33/50 G01N33/68 |
| X | US 2003/003517 A1 (KLEIN ELLIOTT S [US] ET AL) 2 January 2003 (2003-01-02) * the whole document * In particular: paragraphs 8-10, 20 and 26; claims 1-26. ----- | 1-46 | |
| X | WO 02/077229 A (LION BIOSCIENCE AG [DE]; ALBERS MICHAEL [DE]; ELLWANGER SILVIA [DE]; L) 3 October 2002 (2002-10-03) * the whole document * In particular: p. 1, last two paragraphs; p. 3, l. 4 - p. 5, l. 27; p. 24, l. 10-21; p. 25-26, bridging paragraph; p. 27, last two paragraphs; claims 16 and 17. ----- | 1-46 | |

TECHNICAL FIELDS
SEARCHED     (IPC)

G01N

## INCOMPLETE SEARCH

The Search Division considers that the present application, or one or more of its claims, does/do
not comply with the EPC to such an extent that a meaningful search into the state of the art cannot
be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 15 March 2007 | ANGIONI, C |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

...........................................................................

& : member of the same patent family, corresponding
    document

EPO FORM 1503 03.82 (P04C07)

**European Patent Office**

**INCOMPLETE SEARCH
SHEET C**

Application Number

EP 06 02 4330

Claim(s) searched completely:
    1-39,41-46

Claim(s) searched incompletely:
    40

Reason for the limitation of the search:

The present claim 40 relates to an extremely large number of possible compounds. Support and disclosure in the sense of Article 84 and 83 EPC is to be found however for only a very small proportion of the compounds claimed, see p. 28-36. The non-compliance with the substantive provisions is to such an extent, that a meaningful search of the whole claimed subject-matter of the claim could not be carried out (Rule 45 EPC and Guidelines B-VIII, 3). The extent of the search was consequently limited.

European Patent
Office

Application Number

EP 06 02 4330

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-22,25-46 (all in part)

| | | | |
|---|---|---|---|
| **European Patent Office** | **LACK OF UNITY OF INVENTION SHEET B** | Application Number | EP 06 02 4330 |

The Search Division considers that the present European patent application does not comply with the requirements of unity of invention and relates to several inventions or groups of inventions, namely:

Inventions 1-8: Claims 1-22,25-46 (all in part)

Method for determining the effect of a test agent on the liver x receptor alpha, for screening for a test agent which is a modulator for modulating the interaction of the liver x receptor alpha and a co-factor, for determining the presence of a test agent in a sample and provision of a modulator identified by a method of screening, wherein the co-factor is either Tif2, Src1, NcoA3, PGC1, RIP140, NCoR1, SMRT1 or ARNT.

---

Invention 9: Claims 1-22, 25-46 (all in part)

Method for determining the effect of a test agent on a target molecule, for screening for a test agent which is a modulator for modulating the interaction of the target molecule and a target molecule interacting entity, for determining the presence of a test agent in a sample and provision of a modulator identified by a method of screening, wherein the target molecule is the liver x receptor beta.

---

Invention 10: Claims 1-21 (in part), 23 (completely), 25-46 (all in part)

Method for determining the effect of a test agent on a target molecule, for screening for a test agent which is a modulator for modulating the interaction of the target molecule and a target molecule interacting entity, for determining the presence of a test agent in a sample and provision of a modulator identified by a method of screening, wherein the target molecule is a heterodimer of the liver x receptor alpha and the retinoid x receptor alpha.

---

Invention 11: Claims 1-21 (in part), 24 (completely), 25-46 (all in part)

Method for determining the effect of a test agent on a target molecule, for screening for a test agent which is a modulator for modulating the interaction of the target molecule and a target molecule interacting entity, for determining the presence of a test agent in a sample and provision of a modulator identified by a method of screening, wherein the target molecule is the aryl hydrocarbon receptor.

---

**ANNEX TO THE EUROPEAN SEARCH REPORT
ON EUROPEAN PATENT APPLICATION NO.**

EP 06 02 4330

This annex lists the patent family members relating to the patent documents cited in the above-mentioned European search report.
The members are as contained in the European Patent Office EDP file on
The European Patent Office is in no way liable for these particulars which are merely given for the purpose of information.

15-03-2007

| Patent document cited in search report | | Publication date | Patent family member(s) | | Publication date |
|---|---|---|---|---|---|
| WO 02058532 | A | 01-08-2002 | AU | 2002219773 A1 | 06-08-2002 |
| | | | EP | 1355891 A2 | 29-10-2003 |
| | | | JP | 2004523231 T | 05-08-2004 |
| | | | US | 2004077613 A1 | 22-04-2004 |
| US 2003003517 | A1 | 02-01-2003 | WO | 02077169 A2 | 03-10-2002 |
| WO 02077229 | A | 03-10-2002 | AU | 2002304826 A1 | 08-10-2002 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 19742706
- EP 0533838 A

- WO 9808856 A

**Non-patent literature cited in the description**

- Current Protocols in Cell Biology. Wiley InterScience, October 2006
- **MULLER-TAUBENBERGER A.** Application of fluorescent protein tags as reporters in live-cell imaging studies. *Methods Mol Biol.,* 2006, vol. 346, 229-46
- **HASTINGS JW ; MORIN JG.** Photons for reporting molecular events: green fluorescent protein and four luciferase systems. *Methods Biochem Anal.,* 2006, vol. 47, 15-38
- **WILSON T ; HASTINGS JW.** *Bioluminescence. Annu Rev Cell Dev Biol.,* 1998, vol. 14, 197-230

- **WAUGH DS.** Making the most of affinity tags. *Trends Biotechnol.,* 2005, vol. 23, 316-20
- **LICHTY JJ ; MALECKI JL ; AGNEW HD ; MICHELSON-HOROWITZ DJ ; TAN S.** Comparison of affinity tags for protein purification. *Protein Expr Purif.,* 2005, vol. 41, 98-105
- **ALBERS M ; BLUME B ; SCHLUETER T ; WRIGHT ; KOBER I ; KREMOSER C ; DEUSCHLE U ; KOEGL M.** A novel principle for partial agonism of liver X receptor ligands. Competitive recruitment of activators and repressors. *J Biol Chem.,* 2006, vol. 281, 4920-30

SEQUENCE LISTING

<110> PheneX Pharmaceuticals AG

<120> Method for determining the effect of a test agent on a target molecule

<130> P 10014 EP

<160> 3

<170> PatentIn version 3.1

<210> 1
<211> 32
<212> DNA
<213> Artificial

<220>
<221> misc_feature
<223> nucleic acid molecule binding to heterodimer of LXRalpha and RXRalpha

<400> 1
gaaagaaatt tgaccgatag taacctctgc gc                                    32

<210> 2
<211> 38
<212> DNA
<213> Artificial

<220>
<221> misc_feature
<223> primer

<400> 2
gatcgcctcg agacaagttt gtacaaaaaa gctgaacg                              38

<210> 3
<211> 34
<212> DNA
<213> Artificial

<220>
<221> misc_feature
<223> primer

<400> 3
gatcgctcta gaatcaccac tttgtacaag aaag                                  34